# EUROPEAN PATENT APPLICATION

(11) **EP 1 574 515 A2**
(43) Date of publication of application: **14.09.2005**
(21) Application number: 05005276.0
(22) Date of filing: 10.03.2005
(51) Int. Cl.: C07H 3/08, C07D 317/26

(54) **Production method of 2-deoxy-L-ribose compound**

(30) Priority: 12.03.2004 JP 2004071101; 17.09.2004 JP 2004272619
(71) Applicant: Ajinomoto Co., Inc., Tokyo (JP)
(72) Inventor: Oka, Sachiko, Kawasaki-shi Kanagawa 210-8681 (JP); Honda, Yutaka, Kawasaki-shi Kanagawa 210-8681 (JP); Izawa, Kunisuke, Kawasaki-shi Kanagawa 210-8681 (JP)
(74) Representative: Strehl Schübel-Hopf & Partner

(57) **Abstract**

An aldehyde compound represented by the formula (1) is reacted with an organometallic compound represented by the formula (2) to give an alcohol compound represented by the formula (3), which is then subjected to deprotection of a hydroxyl group and production of aldehyde by acid hydrolysis. 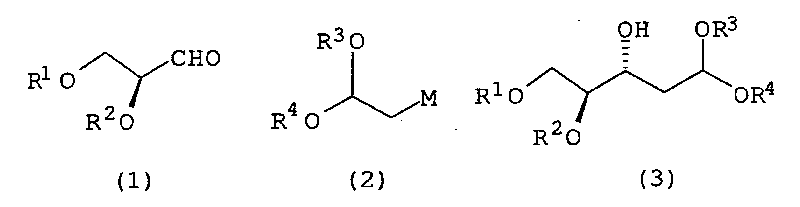 wherein R¹ and R² are each independently a hydroxyl-protecting group or R¹ and R² in combination show a hydroxyl-protecting group, R³ and R⁴ are each independently an alkyl group, an aralkyl group, an aryl group or a silyl group or R³ and R⁴ in combination show a cyclic alkyl group.

## Description

### TECHNICAL FIELD OF THE INVENTION

The present invention relates to a production method of a 2-deoxy-L-ribose compound, namely, 2-deoxy-L-ribose itself and a protected form thereof, and reactive derivatives thereof and the like, and a novel intermediate compound useful for the production of these.

### BACKGROUND OF THE INVENTION

2-Deoxy-L-ribose (L-deoxyribose) is known to be a compound useful as a starting material for the production of an L type nucleic acid derivative that is drawing attention in recent years as a pharmaceutical product such as an antiviral agent and the like (WO 01/034618 and WO 02/044194). In addition, 2-deoxy-L-ribose itself has been reported to have an angiogenesis inhibitory activity (WO 02/051423).

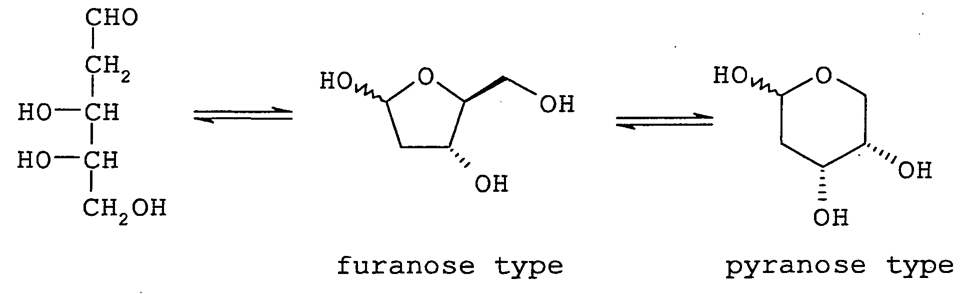

As the production method of 2-deoxy-L-ribose, synthetic methods using L-sugar or D-sugar as a starting material have been generally tried. However, these methods require a great many steps. Particularly, while the use of an L-sugar as a starting material is advantageous for the production of 2-deoxy-L-ribose, which is the same L-sugar, L-sugar scarcely occurs naturally and is difficult to obtain, and known industrially available L-sugar is only L-arabinose. L-Arabinose is a structurally similar L-sugar, which becomes 2-deoxy-L-ribose when the 2-position hydroxyl group is deoxylated. Even when 2-deoxy-L-ribose is produced using this as a starting material, the number of steps is 6 and the total yield is not more than 30% (Tetrahedron Asymmetry, 13, 2667-2672 (2002)).

On the other hand, as a production method that does not use sugar as a starting material, a method using L-glyceraldehyde as a starting material has been reported (Chinese Journal of Chemistry, 20, 1358-1362 (2002)). According to this method, L-glyceraldehyde is propargylated, hydroxyl group is protected, hydrogenation using a Lindlar catalyst and ozone oxidization are performed to give an aldehyde compound, and finally, the protected hydroxyl group is deprotected to synthesize 2-deoxy-L-ribose.

However, according to this method, again, production of 2-deoxy-L-ribose from L-glyceraldehyde requires 5 steps, and this method is not necessarily an efficient production method. In addition, ozone oxidization is not a step suitable for industrial practice, and a production method of 2-deoxy-L-ribose, which is more efficient and suitable for industrial production, has been desired.

### SUMMARY OF THE INVENTION

The present invention aims at provision of an efficient production method suitable for industrial production of 2-deoxy-L-ribose, a protected form thereof and the like, and a novel intermediate compound useful for the production of these.

The present inventors have conducted intensive studies in an attempt to solve the above-mentioned problems and found that 2-deoxy-L-ribose can be obtained by reacting an aldehyde compound represented by the formula (1) to be mentioned later with an organometallic compound represented by the formula (2) to be mentioned later to give an alcohol compound represented by the formula (3) to be mentioned later, and subjecting this compound to deprotection of hydroxyl group and aldehyde production by acid hydrolysis. Furthermore, a hydroxyl group of 2-deoxy-L-ribose can be protected easily to afford a more stable protected form. Moreover, the present inventors have found a method of conveniently purifying a hydroxyl-protected form of the obtained 2-deoxy-L-ribose, which resulted in the completion of the present invention.

That is, the present invention provides the following.
[1] A production method of a 2-deoxy-L-ribose compound, which comprises the following Steps (a) and (b):
   (a) reacting an aldehyde compound represented by the formula (1) : 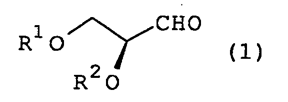 wherein R¹ and R² are each independently a hydroxyl-protecting group or R¹ and R² in combination show a hydroxyl-protecting group, with an organometallic compound represented by the formula (2): 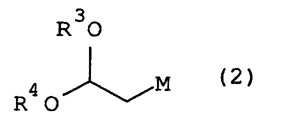 wherein R³ and R⁴ are each independently an alkyl group, an aralkyl group, an aryl group or a silyl group or R³ and R⁴ in combination show a cyclic alkyl group, and M is a metal atom or a metal salt, to give an alcohol compound represented by the formula (3): 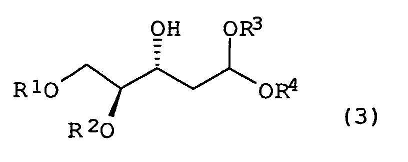 wherein R¹, R², R³ and R⁴ are as defined above, and
   (b) subjecting the alcohol compound represented by the formula (3) to deprotection of the hydroxyl group and production of aldehyde by acid hydrolysis.
[2] The production method of the above-mentioned [1], which affords 2-deoxy-L-ribose.
[3] The production method of the above-mentioned [1], which further comprises Step (c) comprising introducing a protecting group into at least one hydroxyl group of 2-deoxy-L-ribose to give a hydroxyl-protected form of 2-deoxy-L-ribose.
[4] The production method of the above-mentioned [3], wherein, in Step (c), a protecting group is introduced into the 1-position hydroxyl group of 2-deoxy-L-ribose to give a 1-position hydroxyl-protected form of 2-deoxy-L-ribose.
[5] The production method of the above-mentioned [4], wherein, in Step (c), the protecting group of the 1-position hydroxyl group is introduced by the acid hydrolysis in Step (b), which is performed in the co-presence of an acid and an alcohol.
[6] The production method of the above-mentioned [5], wherein the alcohol is methanol.
[7] The production method of any of the above-mentioned [4]-[6], wherein the 1-position hydroxyl-protected form of 2-deoxy-L-ribose is of a furanose type.
[8] A production method of a furanose type 2-deoxy-L-ribose compound represented by the formula (8) 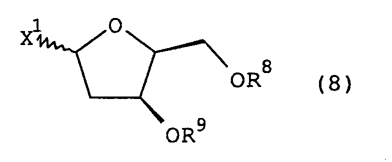 wherein X¹ is a leaving group, R⁸ and R⁹ are each independently a hydroxyl-protecting group or R⁸ and R⁹ in combination show a hydroxyl-protecting group, which comprises protecting the 3-position and the 5-position hydroxyl groups of the furanose type 1-position hydroxyl-protected form of 2-deoxy-L-ribose obtained by the production method of the above-mentioned [7], and then converting the protected 1-position hydroxyl group to a leaving group.
[9] The production method of the above-mentioned [8], wherein X¹ is a chlorine atom and R⁸ and R⁹ are each a p-toluoyl group.
[10] The production method of any of the above-mentioned [1]-[9], wherein R¹ and R² are each independently a hydroxyl-protecting group, which can be deprotected by acid hydrolysis, or R¹ and R² in combination show a hydroxyl-protecting group, which can be deprotected by acid hydrolysis, and the deprotection of the hydroxyl group and the production of aldehyde by acid hydrolysis of Step (b) are simultaneously performed.
[11] The production method of the above-mentioned [10], wherein R¹ and R² in combination show a cyclic acetal type hydroxyl-protecting group, which can be deprotected by acid hydrolysis.
[12] The production method of any of the above-mentioned [1]-[11], wherein M is Li, MgX² or ZnX² (X² is a halogen atom).
[13] The production method of the above-mentioned [11], wherein the aldehyde compound represented by the formula (1) is an aldehyde compound represented by the formula (1-a): 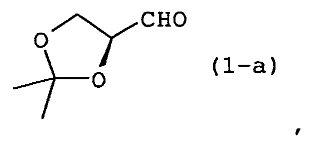 the organometallic compound represented by the formula (2) is an organometallic compound represented by the formula (2-a): 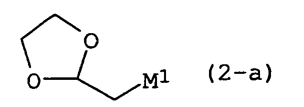 wherein M¹ is Li, MgX² or ZnX² (X² is a halogen atom), and the alcohol compound represented by the formula (3) is an alcohol compound represented by the formula (3-a): 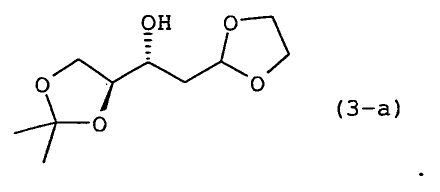
[14] A production method of a furanose type 1-position hydroxyl-protected form of 2-deoxy-L-ribose, which comprises reacting a mixture of a furanose type 1-position hydroxyl-protected form of 2-deoxy-L-ribose and a furanose type 1-position hydroxyl-protected form of 2-deoxy-L-xylose with a ketone compound represented by the formula (5) 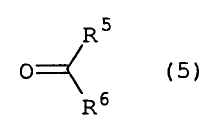 wherein R⁵ and R⁶ are each independently an alkyl group, an aralkyl group or an aryl group, or R⁵ and R⁶ in combination show a cyclic alkyl group, in the presence of an acid, to preferentially protect the 3-position and the 5-position hydroxyl groups of the furanose type 1-position hydroxyl-protected form of 2-deoxy-L-xylose and give a furanose type 2-deoxy-L-xylose compound represented by the formula (6) 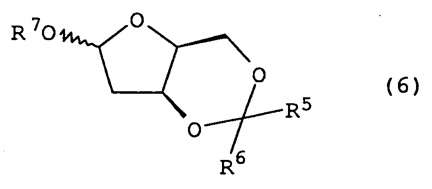 wherein R⁵ and R⁶ are as defined above and R⁷ is an alkyl group or an aralkyl group, and then separating an unreacted furanose type 1-position hydroxyl-protected form of 2-deoxy-L-ribose from the furanose type 2-deoxy-L-xylose compound represented by the aforementioned formula (6).
[15] The production method of the above-mentioned [14], wherein the ketone compound represented by the formula (5) is acetone.
[16] The production method of the above-mentioned [14] or [15], wherein the separation comprises separation of water from an organic solvent.
[17] A production method of a furanose type 2-deoxy-L-ribose represented by the formula (8) 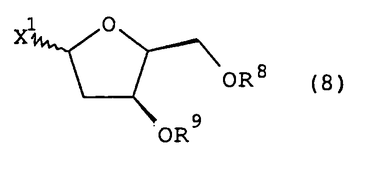 wherein X¹ is a leaving group, R⁸ and R⁹ are each independently a hydroxy-protecting group or R⁸ and R⁹ in combination show a hydroxyl-protecting group, which comprises protecting the 3-position and the 5-position hydroxyl groups of the furanose type 1-position hydroxyl-protected form of 2-deoxy-L-ribose obtained by the production method of any of the above-mentioned [14]-[16], and then converting the protected 1-position hydroxyl group to a leaving group.
[18] The production method of the above-mentioned [17], wherein X¹ is a chlorine atom and R⁸ and R⁹ are p-toluoyl groups. [19] The production method of any of the above-mentioned [14]-[18], wherein the mixture containing a furanose type 1-position hydroxyl-protected form of 2-deoxy-L-ribose and a furanose type 1-position hydroxyl-protected form of 2-deoxy-L-xylose are obtained by the production method of any of the above-mentioned [4]-[6].
[20] The production method of the above-mentioned [19], wherein R¹ and R² are each independently a hydroxyl-protecting group, which can be deprotected by acid hydrolysis, or R¹ and R² in combination show a hydroxyl-protecting group, which can be deprotected by acid hydrolysis, and the deprotection of the hydroxyl group and the production of aldehyde by acid hydrolysis of Step (b) are simultaneously performed.
[21] The production method of the above-mentioned [20], wherein R¹ and R² in combination show a cyclic acetal type hydroxyl-protecting group, which can be deprotected by acid hydrolysis.
[22] The production method of any of the above-mentioned [19]-[21], wherein M is Li, MgX² or ZnX² (X² is a halogen atom).
[23] The production method of the above-mentioned [21], wherein the aldehyde compound represented by the formula (1) is an aldehyde compound represented by the formula (1-a): 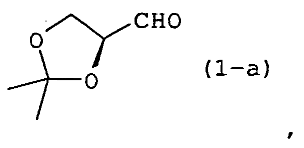 the organometallic compound represented by the formula (2) is an organometallic compound represented by the formula (2-a): 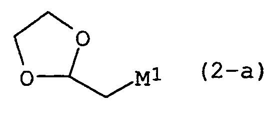 wherein M¹ is Li, MgX² or ZnX² (X² is a halogen atom), and the alcohol compound represented by the formula (3) is an alcohol compound represented by the formula (3-a): 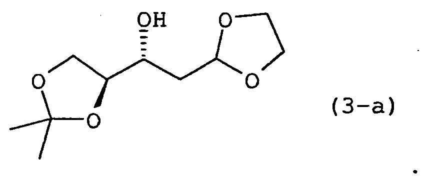
[24] An alcohol compound represented by the formula (3): 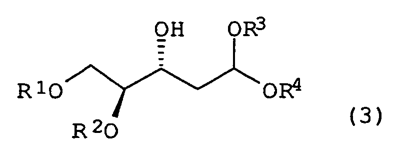 wherein R¹ and R² are each independently a hydroxyl-protecting group or R¹ and R² in combination show a hydroxyl-protecting group, R³ and R⁴ are each independently an alkyl group, an aralkyl group, an aryl group or a silyl group or R³ and R⁴ in combination show a cyclic alkyl group.
[25] An alcohol compound represented by the formula (3-a):

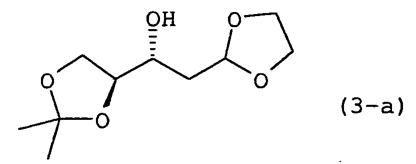

According to the present invention, 2-deoxy-L-ribose and a protected form thereof can be efficiently produced by a small number of steps, and this method is an industrially suitable method.

### DETAILED DESCRIPTION OF THE INVENTION

The 2-deoxy-L-ribose compound of the present invention encompasses the 2-deoxy-L-ribose itself and a hydroxyl-protected form thereof.

The production method of the 2-deoxy-L-ribose compound of the present invention essentially comprises Steps (a) and (b).

In Step (a), an aldehyde compound represented by the formula (1) : 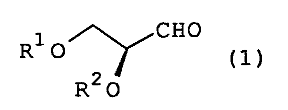 wherein R¹ and R² are each independently a hydroxyl-protecting group or R¹ and R² in combination show a hydroxyl-protecting group is reacted with an organometallic compound represented by the formula (2): 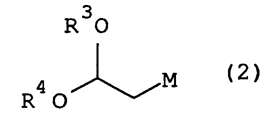 wherein R³ and R⁴ are each independently an alkyl group, an aralkyl group, an aryl group or a silyl group or R³ and R⁴ in combination show a cyclic alkyl group, M is a metal atom or a metal salt to give an alcohol compound represented by the formula (3): 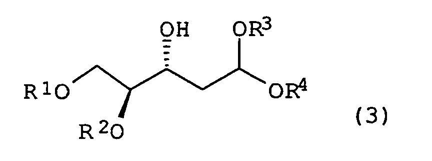 wherein R¹, R², R³ and R⁴ are as defined above.

The aldehyde compound represented by the formula (1) can be produced easily by converting an amino group of L-serine to a hydroxyl group, esterifying a carboxyl group, protecting the hydroxyl group to synthesize methyl 2,3-O-protected-L-glycerate (see Helvetica Chimica Acta, vol. 68, pp. 1863-1871 (1985) etc.), and reducing this compound to give a 2,3-O-protected-L-glyceraldehyde compound.

The organometallic compound represented by the formula (2) can be synthesized easily by halogenation of vinyl acetate as a starting material in a methanol solution to give a dimethyl acetal of halogenated acetaldehyde, which is followed by conversion of the protecting group to give a protected form of halogenated acetaldehyde (see GB patent publication No. 2146016), which is then reacted with metal to give the object compound. It can be also synthesized by synthesizing protected acetaldehyde using acetaldehyde as a starting material and halogenating the same (see WO99/217346 and EP-A-413558 etc.).

The hydroxyl-protecting group for R¹ or R² is not particularly limited as long as it is a hydroxyl-protecting group and, for example, cyclic acetal type protecting groups such as methoxymethylene acetal group, isopropylidene group, methylene acetal group, ethylidene acetal group, methyl group, benzyl group, benzoyl group, t-butyl group, acetyl group, methoxymethyl group, 2-methoxyethoxymethyl group, trimethylsilyl group, triethylsilyl group, t-butyldimethylsilyl group, methylthiomethyl group, trifluoroacetyl group, benzyloxycarbonyl group, t-butoxycarbonyl group, triphenylmethyl group, toluoyl group, 4-methoxymethylphenyl group and the like can be mentioned.

As the hydroxyl-protecting group for R¹ or R², a hydroxyl-protecting group, which can be deprotected by acid hydrolysis, is preferable. As the hydroxyl-protecting group, which can be deprotected by acid hydrolysis, for example, cyclic acetal type protecting groups such as methoxymethylene acetal group, isopropylidene group, methylene acetal group and ethylidene acetal group, methoxymethyl group, trimethylsilyl group, triethylsilyl group, t-butyldimethylsilyl group, methylthiomethyl group, benzyloxycarbonyl group, t-butoxycarbonyl group, triphenylmethyl group and the like can be mentioned.

As R¹ or R², from the aspect of the stereoselectivity of the reaction, cyclic acetal type protecting groups such as methoxymethylene acetal group, isopropylidene group, methylene acetal group and ethylidene acetal group are preferable, which are formed by R¹ and R² in combination, and an isopropylidene group is particularly preferable.

As the "alkyl group" for R³ or R⁴, a linear or branched chain alkyl group having preferably 1-20, more preferably 1-7, carbon atoms can be mentioned. Specifically, for example, alkyl group such as methyl group, ethyl group, n-propyl group, isopropyl group, n-butyl group, isobutyl group, sec-butyl group, tert-butyl group, lauryl group and the like can be mentioned.

The alkyl group is optionally substituted by one or more substituents below. As the substituent used herein, for example, a linear or branched chain alkoxy group (preferable carbon number: 1-6, e.g.: methoxy group and the like), a halogen atom (e.g., chlorine atom, fluorine atom and the like), a hydroxyl group and the like can be mentioned. It is needless to say that the "alkyl group having substituent" is encompassed in the "alkyl group" in the present invention.

The "aralkyl group" for R³ or R⁴ is one wherein the aryl moiety preferably has 6-12, more preferably 6-8, carbon atoms, and the alkyl moiety is a linear or branched chain alkyl group preferably having 1-6, more preferably 1-3, carbon atoms. Specific examples of the aralkyl group preferably include a benzyl group.

The aralkyl group is optionally substituted by one or more substituents below. As the substituent here, for example, a nitro group, a linear or branched chain alkoxy group (preferable carbon number: 1-6, e.g.: methoxy group and the like), a halogen atom (e.g., chlorine atom, fluorine atom and the like), a hydroxyl group and the like can be mentioned. It is needless to say that the "aralkyl group having substituent" is encompassed in the "aralkyl group" in the present invention.

As the "aryl group" for R³ or R⁴, an aryl group preferably having 6-20, more preferably 6-8, carbon atoms can be mentioned. The aryl group is optionally substituted by one or more substituents below. As the substituent here, for example, a nitro group, a linear or branched chain alkoxy group (preferable carbon number: 1-6, e.g.: methoxy group and the like), a halogen atom (e.g., chlorine atom, fluorine atom and the like), a linear or branched chain alkyl group (preferable carbon number: 1-4, e.g.: methyl group, ethyl group, propyl group and the like), a hydroxyl group and the like can be mentioned. It is needless to say that the "aryl group having substituent" is encompassed in the "aryl group" in the present invention. Specific examples of the aryl group optionally having substituents include a phenyl group, o-, m- or p-nitrophenyl group, o-, m- or p-methoxyphenyl group, o-, m- or p-chlorophenyl group, o-, m- or p-fluorophenyl group, o-, m- or p-tolyl group and the like.

As the "silyl group" for R³ or R⁴, for example, a trimethylsilyl group, a triethylsilyl group, triisopropylsilyl and a t-butyldimethylsilyl group can be mentioned. Particularly, a t-butyldimethylsilyl group is preferable.

When R³ and R⁴ in combination show a cyclic alkyl group, a cyclic alkyl group having 2-6 carbon atoms is preferable. As used herein, the "cyclic alkyl group" means an "alkylene group". The cyclic alkyl group is optionally substituted by one or more substituents below. As the substituent here, for example, a nitro group, a linear or branched chain alkoxy group (preferable carbon number: 1-6, e.g., methoxy group and the like), a halogen atom (e.g., chlorine atom, fluorine atom and the like), a linear or branched chain alkyl group (preferable carbon number: 1-4, e.g., methyl group, ethyl group, propyl group and the like), a hydroxyl group and the like can be mentioned. It is needless to say that the "cyclic alkyl group having substituent" is encompassed in the "cyclic alkyl group" in the present invention.

As the metal atom for M, lithium atom (Li), sodium atom (Na), potassium atom (K) and the like can be mentioned. As the metal salt for M, magnesium halide (MgX²), zinc halide (ZnX²) and the like can be mentioned. As M, lithium atom, magnesium halide (MgX²) and zinc halide (ZnX²) are particularly preferable. Here, X² means a halogen atom, and as the halogen atom, bromine atom, chlorine atom and iodine atom are preferable, and bromine atom is particularly preferable.

As the aldehyde compound represented by the formula (1), an aldehyde compound represented by the formula (1-a) 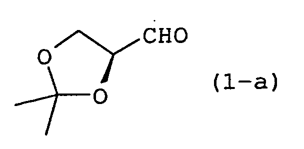 wherein a hydroxyl group is protected by an isopropylidene group, is preferable in view of the stereoselectivity of the reaction and comparatively easy production and availability. As the organometallic compound represented by the formula (2), an organometallic compound represented by the following formula (2-a) 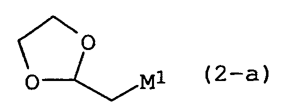 wherein M¹ is Li, MgX² or ZnX², wherein X² is a halogen atom and R³ and R⁴ in combination show a cyclic alkyl group having 2 carbon atoms, is particularly preferable, in view of comparatively easy production and availability.

As the halogen atom shown by X², a chlorine atom, a bromine atom and an iodine atom are preferable, and bromine atom is particularly preferable.

Of the organometallic compounds represented by the formula (2-a), an organometallic compound represented by the following formula (2-b) is preferable.

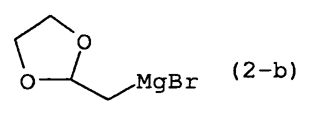

The organometallic compound represented by the formula (2) is used at a ratio of generally 0.5 molar equivalent - 2 molar equivalents, preferably 1 molar equivalent - 1.5 molar equivalents, relative to an aldehyde compound represented by the formula (1). The reaction is generally carried out in a suitable organic solvent. The organic solvent to be used is not particularly limited as long as it is inactive to the reaction and, for example, ethers such as diethyl ether, diisopropyl ether, dimethyl ether, t-butyl methyl ether, dibutyl ether, petroleum ether and the like, cyclic ethers such as tetrahydrofuran (THF), dioxane and the like, benzene, toluene, xylene, butane, pentane, hexane, heptane and the like can be mentioned. Particularly, tetrahydrofuran and diethyl ether are preferable. These organic solvents are preferably used after dehydration and purge with an inactive gas. The amount of the solvent to be used is not particularly limited but generally used at a weight ratio of 0.5- to 200-fold, preferably 1- to 10-fold, relative to an aldehyde compound.

The reaction temperature is generally -78°C to 100°C, preferably -30°C to 66°C. The reaction time is not particularly limited but generally 10 min - 24 hr, preferably 30 min - 5 hr, in the above-mentioned temperature range.

In Step (a), an organometallic compound represented by the formula (2) may be prepared in a reaction vessel by a reaction with a suitable reagent. For example, 2-bromomethyl-1,3-dioxolane is reacted with magnesium in a reaction vessel to give (1,3-dioxolan-2-ylmethyl)-magnesium bromide. Similarly, 2-bromomethyl-1,3-dioxolane is reacted with zinc to give (1,3-dioxolan-2-ylmethyl)-zinc bromide. In addition, 2-bromomethyl-1,3-dioxolane is reacted with lithium diisopropylamide to give (1,3-dioxolan-2-ylmethyl)-lithium.

After the completion of the reaction, an alcohol compound represented by the formula (3) is isolated from the reaction solution by a method known to those of ordinary skill in the art, such as chromatography and the like. It is also possible to use the reaction solution as it is for the next step without isolation, and, where necessary, a different solvent may be added after concentration or evaporation of the reaction solution and used for the next step.

As for the alcohol compound represented by the formula (3), which is obtained in Step (a), it is possible to highly stereoselectively obtain one having a steric configuration of the 3-position R form. In this case, the 3-position S form, which is generated as a minor component, can be removed by chromatography. However, in view of the fact that the 3-position R form and the 3-position S form are diastereomers having similar properties and industrial purification thereof by chromatography is practically difficult, without performing purification at this stage , it is preferable to remove a byproduct derived from the 3-position S form in Step (d) to be mentioned later.

An embodiment of the synthesis of an alcohol compound, which comprises reacting 2,3-O-isopropylidene-D-glyceraldehyde, which is an optical isomer of an aldehyde compound represented by the formula (1-a) in the present invention, with (1,3-dioxolan-2-ylmethyl)-magnesium bromide is described in WO 03/000200 (Fig. 1 and Example 1 on page 66). However, production of a compound having the same steric configuration as the alcohol compound represented by the formula (3) has not been known. In addition, WO 03/000200 does not describe stereoselectivity of the above-mentioned reaction and it was first found in the present invention that a compound represented by the formula (3), which has a 3-position steric configuration of R, can be obtained stereoselectively. In this reference, moreover, the obtained alcohol compound is oxidized to convert to a carbonyl group and therefore, it is clear that this invention does not aim at production of 2-deoxyribose utilizing stereoselectivity of alcohol compound as in the present invention.

Step (b) comprises subjecting an alcohol compound represented by the formula (3) to elimination of a hydroxyl-protecting group and production of aldehyde by acid hydrolysis. An alcohol compound represented by the formula (3) can be led to 2-deoxy-L-ribose by eliminating a hydroxyl-protecting group represented by R¹ or R² to give a hydroxyl group, and hydrolyzing a group represented by R³ or R⁴ with an acid to give an aldehyde group.

When the hydroxyl-protecting group represented by R¹ or R² is a hydroxyl-protecting group, which can be deprotected by acid hydrolysis, deprotection of the hydroxyl group and generation of aldehyde can be simultaneously performed by acid hydrolysis and 2-deoxy-L-ribose can be produced in a single step. When a hydroxyl-protecting group represented by R¹ or R² cannot be removed by acid hydrolysis, deprotection of the hydroxyl group is performed independently of the aldehyde producing reaction. This deprotection is preferably performed before aldehyde producing reaction. Deprotection of the hydroxyl group is performed by a method known to those of ordinary skill in the art.

As the acid to be used in Step (b), acetic acid, trifluoroacetic acid, hydrochloric acid, sulfuric acid, strong acidic resin and the like can be mentioned, and acetic acid and hydrochloric acid are particularly preferable. The amount of the acid to be used is not particularly limited, but it is generally preferably 0.1 molar equivalent to 200 molar equivalents, preferably 1 molar equivalent to 50 molar equivalents, relative to an alcohol compound represented by the formula (3).

The acid hydrolysis can be performed by, for example, dropwise addition of an acid to a solvent in which an alcohol compound represented by the formula (3) has been dissolved. In this case, it is preferable to dissolve an alcohol compound represented by the formula (3) in an organic solvent immiscible with water and dropwise add an aqueous acid solution, so as to suppress side reaction by the acid. As preferable organic solvent, methylene chloride, chloroform, tetrahydrofuran and the like can be mentioned. Methylene chloride is particularly preferable. The amount of the organic solvent to be used is not particularly limited but generally used in a weight ratio of 1- to 200-fold, preferably 10- to 50-fold, relative to an alcohol compound represented by the formula (3). While the concentration of the aqueous acid solution is not particularly limited, either, the acid is generally used in a volume ratio of 0.1-100%, preferably 5-80%, of the water.

The reaction temperature of the acid hydrolysis is generally 0°C-100°C, preferably 10°C-50°C. The reaction time is not particularly limited but the reaction completes in the above-mentioned temperature range generally for 10 min-6 hr, preferably 30 min-2 hr.

After the completion of the reaction, 2-deoxy-L-ribose in the reaction solution is isolated by a method known to those of ordinary skill in the art, such as chromatography and the like. In addition, the reaction solution may be used as it is in the next step without isolation, and where necessary, the reaction solution may be concentrated or evaporated, added with a different solvent and used in the next step.

In Step (c), at least one hydroxyl group of 2-deoxy-L-ribose obtained in Step (b) is protected to give a hydroxyl-protected form of 2-deoxy-L-ribose. This step is optionally employed when producing a protected form of 2-deoxy-L-ribose.

In Step (c), a hydroxyl group of 2-deoxy-L-ribose can be protected by a method known to those of ordinary skill in the art. As the protecting group, those known as a hydroxyl-protecting group can be used, such as methyl group, benzyl group, 4-methoxymethylphenyl group, methoxymethyl group, trimethylsilyl group, triethylsilyl group, t-butyldimethylsilyl group, acetyl group, benzoyl group, toluoyl group, isopropylidene group, methylthiomethyl group, 2-methoxyethoxymethyl group, trifluoroacetyl group, t-butyl group, benzyloxycarbonyl group, t-butoxycarbonyl group, triphenylmethyl group and the like.

In the acid hydrolysis of Step (b), the 1-position hydroxyl group is selectively protected in the co-presence of an acid and an alcohol, whereby Step (c) can be performed simultaneously. In other words, a 1-position hydroxyl-protected form of 2-deoxy-L-ribose, wherein the 1-position hydroxyl group is protected by an alkyl group corresponding to the alcohol used, can be produced in a single step. The 1-position hydroxyl-protected form can fix the skeleton of 2-deoxy-L-ribose in an equilibrium state. Therefore, it is advantageous for isolation and purification and derivaritization thereafter. As preferable alcohol, alcohol having 1-8 carbon atoms can be mentioned. To be specific, alcohols such as methanol, ethanol, isopropyl alcohol, butyl alcohol, benzyl alcohol and the like can be mentioned, with particular preference given to methanol. The amount of the alcohol to be used is not particularly limited, but generally used in a weight ratio of 1- to 200-fold, preferably 5- to 50-fold, relative to an alcohol compound represented by the formula (3).

When R⁷OH is the alcohol used, the thus-obtained 1-position hydroxyl-protected form of 2-deoxy-L-ribose is generally obtained as a mixture of a furanose type represented by the following formula (4) and a pyranose type represented by the formula (4'). As a starting material of a pharmaceutical product, a furanose type is preferable, wherein R⁷ is an alkyl group or an aralkyl group. An alkyl group and an aralkyl group having 1-8 carbon atoms are preferable. As the specific alcohol, those mentioned above can be mentioned.

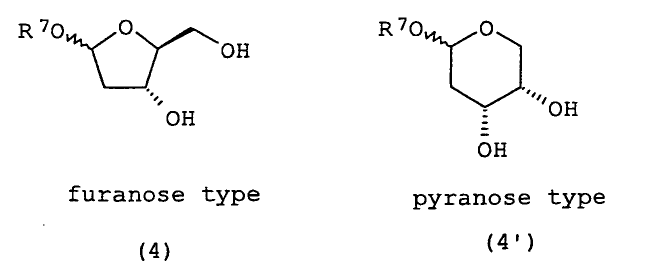

After the completion of the reaction of Step (c), a hydroxyl-protected form of 2-deoxy-L-ribose is isolated from the reaction solution by a method known to those of ordinary skill in the art such as chromatography and the like. When the object product of Step (c) is the above-mentioned furanose type 1-position hydroxyl-protected form of 2-deoxy-L-ribose represented by the formula (4) (hereinafter to be also referred to as compound (4)), and particularly when the furanose type 1-position hydroxyl-protected form of 2-deoxy-L-xylose represented by the following formula (4-a) (hereinafter to be also referred to as compound (4-a)) derived from the 3-position S form of an alcohol compound represented by the formula (3) by-produced in the above-mentioned Step (a) is mixed in the reaction mixture, compound (4) can be efficiently isolated by performing the following Step (d).

As shown in the following scheme, in Step (d), a mixture containing compound (4) and compound (4-a) is reacted with a ketone compound represented by the formula (5) (hereinafter to be also referred to as ketone compound (5)) in the presence of an acid to preferentially protect the 3-position and the 5-position hydroxyl groups of compound (4-a) to give a furanose type 2-deoxy-L-xylose compound represented by the formula (6) (hereinafter to be also referred to as compound (6)), and then unreacted compound (4) and compound (6) are separated. 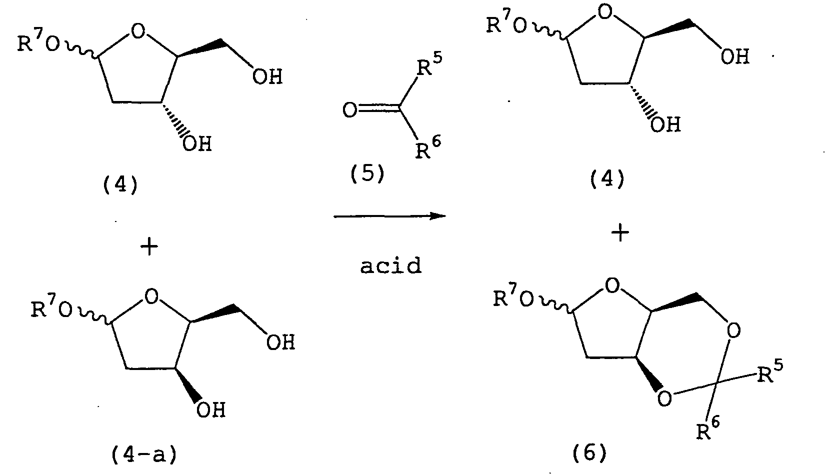 wherein R⁵ and R⁶ are each independently an alkyl group, an aralkyl group or an aryl group, or R⁵ and R⁶ in combination show a cyclic alkyl group and R⁷ is an alkyl group or an aralkyl group.

Since compound (4) and compound (4-a) generally do not have high crystallinity, chromatography is necessary for separation. However, since compound (4) and compound (4-a) are diastereomers and have markedly similar properties, industrial practice of purification by chromatography is difficult to achieve.

When a mixture containing compound (4) and compound (4-a) is reacted with a ketone compound (5) in the presence of an acid in Step (d), compound (6) is easily obtained, since the 3-position and the 5-position hydroxyl groups of compound (4-a) are in the cis-position relative to the furanose ring, and an acetal type protecting group with ketone compound (5) is formed sterically advantageously. In contrast, the 3-position and the 5-position hydroxyl groups of compound (4) is in the transposition relative to the furanose ring, and formation of an acetal-protecting group is sterically disadvantageous. Therefore, compound (4-a) alone can be preferentially protected in Step (d), and a mixture containing compound (4) and compound (6) can be obtained. Since compound (6) has considerably high lipid solubility as compared to compound (4), it can be separated easily from compound (4) by an industrially practical and convenient operation.

The 3-position and the 4-position hydroxyl groups of the pyranose type 2-deoxy-L-ribose represented by the formula (4') are also considered to be easily protected in Step (d). The protected form thereof is also advantageously separated from compound (4) along with compound (6) and other lipid soluble byproducts can be also removed by Step (d).

The "alkyl group" for R⁵ or R⁶ is a linear or branched chain alkyl group preferably having 1-20, more preferably 1-7, carbon atoms. Specifically, for example, alkyl groups such as methyl group, ethyl group, n-propyl group, isopropyl group, n-butyl group, isobutyl group, sec-butyl group, tert-butyl group, lauryl group and the like can be mentioned.

The alkyl group is optionally substituted by the one or more substituents below. As the substituent here, for example, linear or branched chain alkoxy groups (preferable carbon number: 1-6, e.g. : methoxy group and the like), halogen atoms (e.g.,: chlorine atom, fluorine atom and the like), hydroxyl group and the like can be mentioned. It is needless to say that the "alkyl group having substituent" is encompassed in the "alkyl group" in the context of the present invention.

As the "aralkyl group" for R⁵ or R⁶, is an aralkyl group wherein the aryl moiety preferably has 6-12, more preferably 6-8, carbon atoms and the alkyl moiety is a linear or branched chain alkyl group preferably having 1-6, more preferably 1-3, carbon atoms.

The aralkyl group is optionally substituted by one or more substituents below. As the substituent here, for example, a nitro group, a linear or branched chain alkoxy group (preferable carbon number: 1-6, e.g.: methoxy group and the like), halogen atoms (e.g.,: chlorine atom, fluorine atom and the like), hydroxyl groups and the like can be mentioned. It is needless to say that the "aralkyl group having substituent" is encompassed in the "aralkyl group of the present invention". Specific examples of the aralkyl group include substituted benzyl group such as p-methoxybenzyl group, dimethoxybenzyl group and the like and a benzyl group.

As the "aryl group" for R⁵ or R⁶, an aryl group preferably having 6-20, more preferably 6-8, carbon atoms can be mentioned. The aryl group is optionally substituted by one or more substituents below. As the substituent here, for example, nitro group, linear or branched chain alkoxy group (preferable carbon number: 1-6, e.g.: methoxy group and the like), halogen atom (e.g.: chlorine atom, fluorine atom and the like), linear or branched chain alkyl group (preferable carbon number: 1-4, e.g.: methyl group, ethyl group, propyl group and the like), hydroxyl group and the like can be mentioned. It is needless to say that the "aryl group having substituent" is encompassed in the "aryl group of the present invention". Specific examples of the aryl group optionally having substituents include phenyl group, o-, m- or p-nitrophenyl group, o-, m- or p-methoxyphenyl group, o-, m- or p-chlorophenyl group, o-, m- or p-fluorophenyl group, o-, m- or p-tolyl group and the like.

When R⁵ and R⁶ in combination show a cyclic alkyl group, a cyclic alkyl group having 2-6 carbon atoms is preferable. As used herein, the "cyclic alkyl group" means an "alkylene group". The cyclic alkyl group is optionally substituted by one or more substituents below. As the substituent here, for example, a nitro group, a linear or branched chain alkoxy group (preferable carbon number: 1-6, e.g.: methoxy group and the like), halogen atom (e.g.,: chlorine atom, fluorine atom and the like), linear or branched chain alkyl group (preferable carbon number: 1-4, e.g.: methyl group, ethyl group, propyl group and the like), hydroxyl group and the like can be mentioned. It is needless to say that the "cyclic alkyl group having substituent" is encompassed in the "cyclic alkyl of the present invention".

As R⁵ and R⁶, an alkyl group is preferable and a methyl group is more preferable. That is, ketone compound (5) is preferably acetone.

The "alkyl group" and "aralkyl group" for R⁷ are as defined above, and are preferably a methyl group.

The ketone compound (5) is used in an amount of generally 0.5 molar equivalent to 10 molar equivalents, preferably 0.8 molar equivalent to 2 molar equivalents, relative to compound (4-a). When the amount of the ketone compound (5) to be used is greater than this range, compound (4) is also likely to be protected, which is not preferable.

As the acid to be used in Step (d), an acid use as an acid catalyst in an acetalized reaction can be mentioned. Specifically, mineral acids such as hydrochloric acid, sulfuric acid, phosphoric acid and the like, organic acids such as methanesulfonic acid, benzenesulfonic acid, p-toluenesulfonic acid, pyridinium p-toluenesulfonate, camphorsulfonic acid, trifluoroacetic acid and the like, Lewis acid such as boron trifluoride etherate, aluminum trichloride, tin tetrachloride, titanium tetrachloride and the like, strong acidic ion exchange resin, and the like can be mentioned. Particularly, p-toluenesulfonic acid is preferable. The amount of the acid to be used is not particularly limited, but it is preferably used in an amount of generally 0.001 molar equivalent to 1 molar equivalent, preferably 0.01 molar equivalent to 0.2 molar equivalent, relative to compound (4-a). When the amount of the acid to be used is greater than this range, compound (4) unpreferably is likely to be protected.

While the reaction may be carried out in a suitable organic solvent, it is preferably carried out without solvent. When it is carried out in an organic solvent, the organic solvent to be used is not particularly limited as long as it is inert to the reaction. For example, esters such as ethyl acetate and the like, aromatic hydrocarbons such as toluene and the like, halogenated hydrocarbons such as methylene chloride, chloroform and the like are preferable. These organic solvents are preferably subjected to dehydration and purge with an inert gas before use.

The reaction temperature of Step (d) is generally 5°C-100°C, preferably 10°C-50°C. When the reaction temperature is higher than this range, compound (4) may be also protected. The reaction time is not particularly limited and the time is appropriately determined by monitoring the reaction until almost complete disappearance of compound (4-a) by TLC and the like. For example, the reaction time is generally 30 min-24 hr, preferably 1 hr-6 hr, in the above-mentioned temperature range.

After the completion of the reaction, neutralization is performed as necessary to quench the reaction, whereby a mixture containing unreacted compound (4) and compound (6) can be obtained. From this mixture, compound (4), compound (6) and other lipid soluble contaminants can be easily separated by an industrially practical and convenient method (e.g., chromatography etc.). As an industrially preferable method, separation by partitioning between water and a suitable organic solvent can be mentioned.

In other words, since compound (4) has a considerably high water solubility, it is easily partitioned into an aqueous layer by the above-mentioned partitioning operation, compound (6) and other lipid soluble contaminants are easily partitioned into an organic layer, they can be separated easily.

The partitioning operation can be performed by sufficiently stirring a mixture containing compound (4) and compound (6) in water and an organic solvent and standing the mixture to allow phase separation.

The organic solvent to be used for the partitioning operation can be appropriately determined in consideration of the coefficient of partition of compound (4) and the like and, for example, when R⁷ is a methyl group, halogenated hydrocarbons such as methylene chloride, chloroform and the like, esters such as ethyl acetate and the like, ethers such as diethyl ether and the like, and the like can be mentioned, with preference given to methylene chloride.

The ratio of the amounts of water and organic solvent in the partitioning operation can be also appropriately determined in consideration of the coefficient of partition of compound (4) and the like and, for example, when R⁷ is a methyl group and methylene chloride is used as an organic solvent, the volume ratio of water:methylene chloride is preferably 90:10-50:50.

The compound (4) partitioned into the aqueous layer by the partitioning operation can be isolated by a method known to those of ordinary skill in the art. For example, compound (4) is re-extracted with an organic solvent permitting extraction from the aqueous layer, or a method comprising concentrating the aqueous layer and the like can be mentioned.

The furanose type 1-position hydroxyl-protected form of 2-deoxy-L-ribose [compound (4)] obtained by the present invention can be led to a furanose type 2-deoxy-L-ribose compound represented by the formula (8), which is more preferable as an intermediate for a pharmaceutical product, by, as shown in the following reaction scheme, protecting the 3-position and the 5-position hydroxyl groups and converting the protected 1-position hydroxyl group (-OR⁷ in the following formula) to a leaving group: 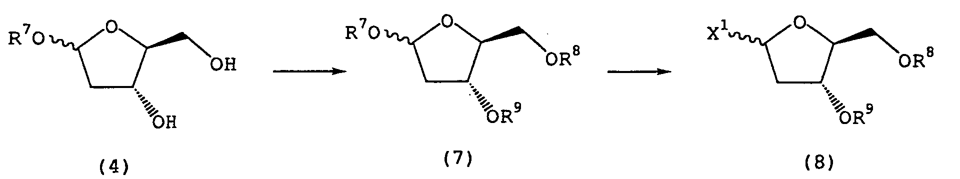 wherein X¹ is a leaving group, R⁸ and R⁹ are each independently a hydroxyl-protecting group or R⁸ and R⁹ in combination show a hydroxyl-protecting group.

The hydroxyl-protecting group for R⁸ or R⁹ is not particularly limited as long as it is a hydroxyl-protecting group and, for example, cyclic acetal type protecting groups such as methoxymethylene acetal group, isopropylidene group, methylene acetal group and ethylidene acetal group, methyl group, benzyl group, benzoyl group, t-butyl group, acetyl group, methoxymethyl group, 2-methoxyethoxymethyl group, trimethylsilyl group, triethylsilyl group, t-butyldimethylsilyl group, methylthiomethyl group, trifluoroacetyl group, benzyloxycarbonyl group, t-butoxycarbonyl group, triphenylmethyl group, toluoyl group, 4-methoxymethylphenyl group and the like can be mentioned.

The leaving group for X¹ is not particularly limited as long as it can leave during, for example, a substitution reaction of a nucleophilic reagent (e.g., nucleic acid basic derivative such as adenine, guanine, 6-chloropurine, uracil, thymine etc.) when a furanose type 2-deoxy-L-ribose compound represented by the formula (8) is leading to a desired compound and, for example, halogen atom (e.g., chlorine atom, bromine atom, iodine atom etc.), sulfonyloxy group (e.g., methanesulfonyloxy group, trifluoromethanesulfonyloxy group, benzenesulfonyloxy group, p-toluenesulfonyloxy group etc.) and the like can be mentioned.

In consideration of the crystallinity of the furanose type 2-deoxy-L-ribose compound represented by the formula (8), R⁸ and R⁹ are preferably p-toluoyl groups, and X¹ is preferably a halogen atom, and more preferably a chlorine atom.

Protection of the 3-position and the 5-position hydroxyl groups of compound (4), and conversion of -OR⁷ to a leaving group can be performed according to a synthetic method of sugar, which is conventionally known to hose of ordinary skill in the art (e.g., Nucleosides & Nucleotides, 18(11 & 12), 2357-2365 (1999)).

For example, a preferable embodiment wherein R⁸ and R⁹ are p-toluoyl groups and X¹ is a chlorine atom, they can be performed according to a method described in Nucleosides & Nucleotides, 18(11 & 12), 2357-2365(1999).

### EXAMPLES

The present invention is explained in detail in the following by referring to Examples, which are not to be construed as limitative.

### Example 1

### Production of 2-deoxy-4,5-O-isopropylidene-L-ribose ethylene acetal

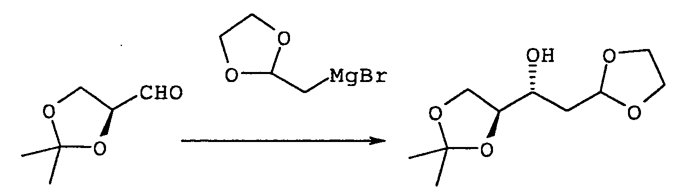

Under an argon atmosphere, a solution (10.8 ml, 0.5M) of (1,3-dioxolan-2-ylmethyl)-magnesium bromide in tetrahydrofuran was added tetrahydrofuran (5 ml), and after stirring, the mixture was cooled to -78°C. Thereto was slowly added a solution of 2,3-O-isopropylidene-L-glyceraldehyde (0.50 g, 3.84 mmol) in tetrahydrofuran (5 ml), and the mixture was stirred for about 2 hr. Thereafter, the temperature was allowed to return to room temperature and the mixture was stirred for 15 hr. After confirmation of complete disappearance of 2,3-O-isopropylidene-L-glyceraldehyde, which is the starting material, by thin layer chromatography (TLC) (hexane:ethyl acetate=1:1), an aqueous ammonium chloride solution was added and the mixture was stirred. The reaction mixture was filtered to remove the salt and extracted with diethyl ether. The organic layer was washed with aqueous sodium chloride solution, dried over sodium sulfate, filtered and concentrated under reduced pressure. The residue was purified by silica gel column chromatography (hexane:ethyl acetate=2:1) to give 2-deoxy-4,5-O-isopropylidene-L-ribose ethylene acetal as a colorless transparent liquid (0.59 g, yield 70.8%, the 3-position R form (deoxyribose): the 3-position S form (deoxyxylose)=4.2:1).
¹H-NMR (CDCl₃, 400MHz) δ 1.36 (s, 3H), 1.42 (s, 3H), 1.77-1.84 (m, 1H), 2.04-2.09(m, 1H), 3.84-4.08 (m, 8H), 5.08(t, 1H, J = 4.6Hz)

### Example 2

### Production of methyl 2-deoxy-L-riboside

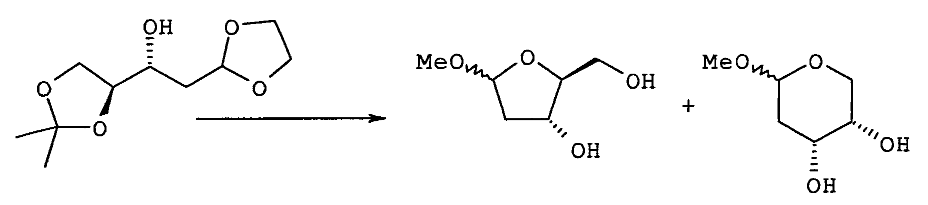

To a solution of 2-deoxy-4,5-O-isopropylidene-L-ribose ethylene acetal (70 mg, 0.32 mmol) in methanol (5 ml) was added 10%-hydrogen chloride-methanol solution (0.1 ml). After dissolution, the mixture was stirred at room temperature for about 4 hr. After confirmation of completion of the reaction by TLC (dichloromethane:methanol=95:5), the mixture was cooled to 0°C and pyridine was added for neutralization (pH 5-6). Thereafter, silica gel was added, and the mixture was concentrated under reduced pressure to remove methanol, whereby the resulting product was adsorbed to silica gel. Thereafter, the product adsorbed to silica gel was purified by silica gel column chromatography (dichloromethane:methanol=98:2) to give methyl 2-deoxy-L-riboside (furanose type:pyranose type=1:4) (41 mg, yield 86.5%).
¹H-NMR(CDCl₃, 400MHz) δ 1.89(dd, 2H, J=2.7Hz, J=4.2Hz), 2.04(d, 1H, J=7.3Hz), 2.21(d, 1H, J=6.0Hz), 3.35(s, 3H), 3.73-3.75(m, 3H), 4.02-4.05(m, 1H), 4.78(dd, 1H, J=2.7Hz, J=3.0Hz), furanose 1-position H(5.12): pyranose 1-position H(4.78)=1:4

### Example 3

### Production of 2-deoxy-L-ribose

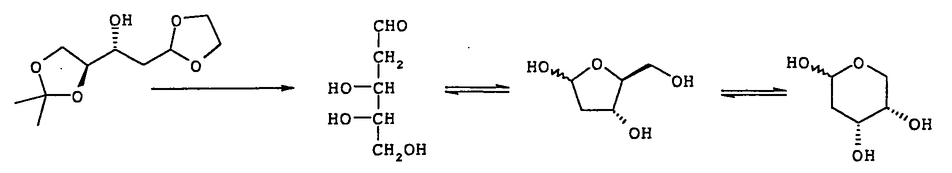

80% Acetic acid (2.5 ml)was added to 2-deoxy-4,5-O-isopropylidene-L-ribose ethylene acetal (50 mg, 0.23 mmol) and the mixture was stirred at room temperature for about 4 hr. After confirmation of completion of the reaction by TLC (hexane:ethyl acetate=1:2), the mixture was concentrated under reduced pressure to remove water. Toluene was added thereto, and the mixture was concentrated under reduced pressure again to remove acetic acid by azeotrope. This step was repeated 3 times to completely remove acetic acid and water. Vacuum drying gave 2-deoxy-L-ribose (30 mg, yield 92.3%).
¹H-NMR (D₂O, 400MHz) δ 1.70-2.40(m, 2H), 3.56-4.25(m, 4H), 5.30(dd, 1H, J=3.3Hz, J=3.0Hz)

### Example 4

To a solution of 2-deoxy-L-ribose (20 mg, 0.14 mmol) obtained in Example 3 in methanol (5 ml) was added 10%-hydrogen chloride-methanol solution (0.05 ml). After dissolution, the mixture was stirred at room temperature for about 2 hr. After confirmation of completion of the reaction by TLC (dichloromethane:methanol=95:5), the mixture was cooled to 0°C and pyridine was added for neutralization (pH 5-6). Thereafter, silica gel was added, methanol was removed by concentration under reduced pressure and the resulting product was adsorbed to silica gel. Thereafter, the product adsorbed to silica gel was purified by silica gel column chromatography (dichloromethane:methanol=98:2) to give methyl 2-deoxy-L-riboside(furanose type: pyranose type =1:2) (18.0 mg, yield 86.9%).
¹H-NMR(CDCl₃, 400MHz) δ 1.89(dd, 2H, J=2.7Hz, J=4.2Hz), 2.04(d, 1H, J=7.3Hz), 2.21(d, 1H, J=6.0Hz), 3.35(s, 3H), 3.73-3.75(m, 3H), 4.02-4.05(m, 1H), 4.78(dd, 1H, J=2.7Hz, J=3.0Hz)

### Reference Example 1

### Production of 2,3-O-isopropylidene-L-glyceraldehyde

Under an argon atmosphere, hexane (39 ml, 0.88M) was added to methyl 2,3-O-isopropylidene-L-glycerate (5 ml, 34.5 mmol), and the mixture was cooled to -78°C. After cooling, diisobutyl aluminum hydride (0.95M hexane solution) (40 ml, 38.0 mmol) was added, and the mixture was stirred at -78°C for 5 hr. After confirmation of completion of the reaction by TLC (hexane:ethyl acetate=2:1), aqueous ammonium chloride solution was added, and the temperature was allowed to return to room temperature. The reaction mixture was filtered and extracted with diethyl ether. The diethyl ether layer was dried over magnesium sulfate, and after filtration, concentrated under reduced pressure. Thereafter, the residue was purified by silica gel column chromatography (hexane:ethyl acetate=3:1) to give 2,3-O-isopropylidene-L-glyceraldehyde as a colorless transparent liquid (3.3 g, yield 89.9%).
¹H-NMR (CDCl₃, 400MHz)δ1.42(s, 3H), 1.49(s, 3H), 4.07-4.20(m, 2H), 4.40(dt, 1H, J=1.8Hz, J=7.0Hz), 9.72(d, 1H, J=1.8Hz)

### Example 5

### Production of 1-(2,2-dimethyl[1,3]dioxolan-4-yl)-2-[1,3]dioxolan-2-ylethanol

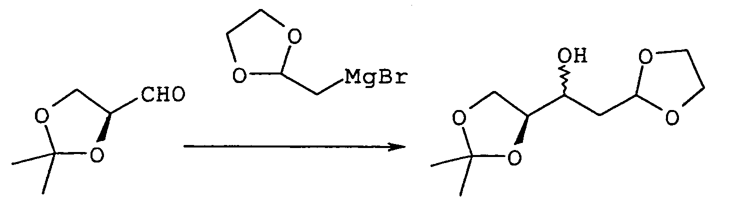

Under an argon atmosphere, to a solution (37 ml, 0.5M) of (1,3-dioxolan-2-ylmethyl)magnesium bromide in tetrahydrofuran was added lithium bromide (3.2 g, 36.9 mmol), and the mixture was stirred at room temperature stirred for 30 min. After stirring, the mixture was cooled to 0°C, and a solution of 2,3-O-isopropylidene-L-glyceraldehyde (2.0 g, 15.4 mmol) in tetrahydrofuran (20 ml) was slowly added thereto. Thereafter, the temperature was raised to 10°C and the mixture was stirred for about 15 hr. After confirmation of complete disappearance of 2,3-O-isopropylidene-L-glyceraldehyde, which was a starting material, by monitoring with thin layer chromatography (TLC) (hexane:AcOEt=1:1), aqueous ammonium chloride solution was added and the mixture was stirred. The reaction mixture was filtered to remove the salt, and the mixture was extracted with diethyl ether. The organic layer was washed with aqueous sodium chloride solution, dried over sodium sulfate, filtered and concentrated under reduced pressure to give 1-(2,2-dimethyl[1,3]dioxolan-4-yl)-2-[1,3]dioxolan-2-ylethanol as an orange viscous liquid (diastereoselectivity; 3R form:3S form=4.5:1), which was used in the next step without purification on the presumption that the yield was 100%.
¹H-NMR(CDCl₃, 400MHz) δ 1.36 (s, 3H), 1.42(s, 3H), 1.77-1.84(m, 1H), 2.04-2.09(m, 1H), 3.84-4.08(m, 8H), 5.08 (t, 1H, J=4.6Hz)

### Example 6

### Syntheses of methyl 2-deoxy-L-riboside and methyl 2-deoxy-L-xyloside

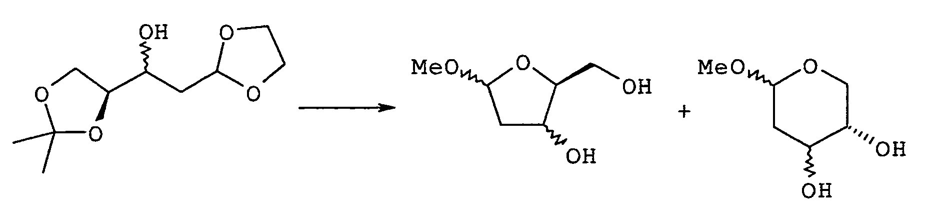

To a solution of 1-(2,2-dimethyl[1,3]dioxolan-4-yl)-2-[1,3]dioxolan-2-ylethanol (15.4 mmol), which was an unpurified orange viscous liquid, in methanol (45 ml) was added 10% hydrogen chloride-methanol (0.46 ml). After dissolution, the mixture was stirred at room temperature for about 3 hr. After confirmation of completion of the reaction by monitoring with TLC (CH₂Cl₂:MeOH=90:10), the mixture was cooled to 0°C, and sodium hydrogen carbonate powder was added for neutralization (pH 6-7). Thereafter, methanol was removed by concentration under reduced pressure to give methyl 2-deoxy-L-riboside (furanose: pyranose=10:1) and methyl 2-deoxy-L-xyloside as an oil (crude, 2.43 g), which was used in the next step without purification on the presumption that the yield was 100%.
¹H-NMR (CDCl₃, 400MHz) δ 2.02-2.30(m, 2H), 3.38 and 3.39 (2s, 3H), 3.60-3.73(m, 2H), 4.03-4.20(m, 1H), 4.50(m, 1H), furanose 1-position H(5.12):pyranose 1-position H(4.78)=10:1

### Example 7

### Synthesis of methyl 2-deoxy-L-riboside

To a solution of methyl 2-deoxy-L-riboside (furanose: pyranose=10:1) and methyl 2-deoxy-L-xyloside (crude, 2.43 g), which was a mixed oil, in acetone (0.5 ml) was added toluenesulfonic acid monohydrate (0.05 g), and the mixture was stirred at room temperature for about 3 hr. After confirmation of completion of the reaction by monitoring with TLC (CH₂Cl₂:MeOH=90:10), aqueous sodium hydrogen carbonate solution was added, 1-O-methyl-3,5-isopropylidene-2-deoxyxylose and other impurities were removed by extraction with dichloromethane. Only the aqueous layer was dried up and the object product was extracted with methanol. Thereafter, the extract was concentrated under reduced pressure to give methyl 2-deoxy-L-riboside as a yellow oil, which was used in the next step without purification on the presumption that the yield was 100%.
¹H-NMR (CDCl₃, 400MHz) δ 2.02-2.30(m, 2H), 3.38 and 3.39(2s, 3H), 3.60-3.73 (m, 2H), 4.03-4.20 (m, 1H), 4.50 (m, 1H), 5.12 (H₁-α) and 5.25 (H₁-β) (t, 1H)

### Example 8

### Synthesis of 1-O-methyl-3,5-ditoluoyl-2-deoxy-L-ribose

To a solution of methyl 2-deoxy-L-riboside (15.4 mmol), which was an unpurified yellow viscous liquid, in acetone (25.6 ml) was added toluoyl chloride (8.0 ml, 61.6 mmol), and triethylamine (9.4 ml, 67.8 mmol) was slowly added dropwise. The mixture was stirred at room temperature for about 20 hr. After confirmation of completion of the reaction by monitoring with TLC (Hexane:AcOEt=4:1), the precipitate was filtered off, and the organic layer was washed with 1N hydrochloric acid, and then with aqueous sodium chloride solution, dried over sodium sulfate, filtered and concentrated under reduced pressure. The filtration and concentration were repeated until the precipitate was not seen, whereby 1-O-methyl-3,5-ditoluoyl-2-deoxy-L-ribose was obtained as a yellow viscous liquid, which was used in the next step without purification on the presumption that the yield was 100%.
¹H-NMR (CDCl₃, 400MHz) δ 2.41 (s, 6H), 3.36 (s, 3H), 3.42 (s, 3H), 4.46-4.65(m, 3H), 5.19(dd, 1H, H₁-α, J=0.95, 5.3Hz), 5.23(dd, 1H, H₁-β, J=2.1, 5.4Hz), 5.41(m, 1H, H₃-α), 5.59 (m, 1H, H₃-β), 7.20-8.05(m, 8H)

### Example 9

### Synthesis of 1-chloro-3,5-ditoluoyl-2-deoxy-α-L-ribose

To a solution of 1-O-methyl-3,5-ditoluoyl-2-deoxy-L-riboside (15.4 mmol), which was an unpurified yellow viscous liquid, in hexane (30.0 ml) was added 4N hydrogen chloride-ethyl acetate (20 ml), and the mixture was stirred at room temperature for about 3.5 hr. After confirmation of completion of the reaction by monitoring with TLC (Hexane:AcOEt=4:1), the mixture was cooled to -5°C and stirred. After a while, crystals were precipitated. The mixture was stirred as it was for about 2 hr to allow sufficient precipitation, and the crystals were then collected by filtration. During collection, the crystals were washed with cold diethyl ether to give 1-chloro-3,5-ditoluoyl-2-deoxy-α-L-ribose as white crystals (0.70 g, yield 11.7% (total yield from 2,3-O-isopropylidene-L-glyceraldehyde)).
¹H-NMR(CDCl₃, 400MHz) δ 2.41 (s, 6H), 2.80 (m, 2H), 4. 64 (m, 2H), 4.86(q, 1H), 5.55 (m, 1H), 6.47(d, 1H, J=5.1Hz), 7.24 (2d, 4H, J=8.6Hz), 7.94 (2d, 4H, J=8.3Hz)

Although only some exemplary embodiments of this invention have been described in detail above, those skilled in the art will readily appreciated that many modifications are possible in the exemplary embodiments without materially departing from the novel teachings and advantages of this invention. Accordingly, all such modifications are intended to be included within the scope of this invention.

## Claims

1. A production method of a 2-deoxy-L-ribose compound, which comprises the following Steps (a) and (b):
(a) reacting an aldehyde compound represented by the formula (1) : 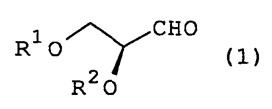 wherein R¹ and R² are each independently a hydroxyl-protecting group or R¹ and R² in combination show a hydroxyl-protecting group, with an organometallic compound represented by the formula (2): 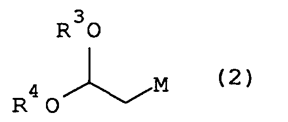 wherein R³ and R⁴ are each independently an alkyl group, an aralkyl group, an aryl group or a silyl group or R³ and R⁴ in combination show a cyclic alkyl group, and M is a metal atom or a metal salt, to give an alcohol compound represented by the formula (3): 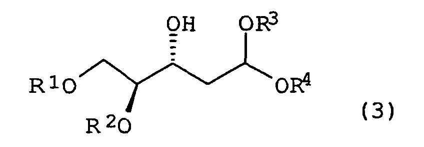 wherein R¹, R², R³ and R⁴ are as defined above, and
(b) subjecting the alcohol compound represented by the formula (3) to deprotection of the hydroxyl group and production of aldehyde by acid hydrolysis.

2. The production method of claim 1, which affords 2-deoxy-L-ribose.

3. The production method of claim 1, which further comprises Step (c) comprising introducing a protecting group into at least one hydroxyl group of 2-deoxy-L-ribose to give a hydroxyl-protected form of 2-deoxy-L-ribose.

4. The production method of claim 3, wherein, in Step (c), a protecting group is introduced into the 1-position hydroxyl group of 2-deoxy-L-ribose to give a 1-position hydroxyl-protected form of 2-deoxy-L-ribose.

5. The production method of claim 4, wherein, in Step (c), the protecting group of the 1-position hydroxyl group is introduced by the acid hydrolysis in Step (b), which is performed in the co-presence of an acid and an alcohol.

6. The production method of claim 5, wherein the alcohol is methanol.

7. The production method of any of claims 4 to 6, wherein the 1-position hydroxyl-protected form of 2-deoxy-L-ribose is of a furanose type.

8. A production method of a furanose type 2-deoxy-L-ribose compound represented by the formula (8) 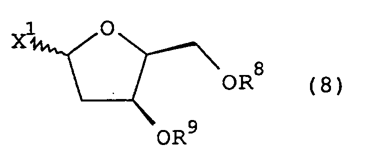 wherein X¹ is a leaving group, R⁸ and R⁹ are each independently a hydroxyl-protecting group or R⁸ and R⁹ in combination show a hydroxyl-protecting group, which comprises protecting the 3-position and the 5-position hydroxyl groups of the furanose type 1-position hydroxyl-protected form of 2-deoxy-L-ribose obtained by the production method of claim 7, and then converting the protected 1-position hydroxyl group to a leaving group.

9. The production method of claim 8, wherein X¹ is a chlorine atom and R⁸ and R⁹ are each a p-toluoyl group.

10. The production method of any of claims 1 to 9, wherein R¹ and R² are each independently a hydroxyl-protecting group, which can be deprotected by acid hydrolysis, or R¹ and R² in combination show a hydroxyl-protecting group, which can be deprotected by acid hydrolysis, and the deprotection of the hydroxyl group and the production of aldehyde by acid hydrolysis of Step (b) are simultaneously performed.

11. The production method of claim 10, wherein R¹ and R² in combination show a cyclic acetal type hydroxyl-protecting group, which can be deprotected by acid hydrolysis.

12. The production method of any of claims 1 to 11, wherein M is Li, MgX² or ZnX² (X² is a halogen atom).

13. The production method of claim 11, wherein the aldehyde compound represented by the formula (1) is an aldehyde compound represented by the formula (1-a): 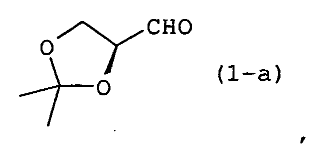 the organometallic compound represented by the formula (2) is an organometallic compound represented by the formula (2-a): 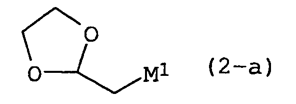 wherein M¹ is Li, MgX² or ZnX² (X² is a halogen atom), and the alcohol compound represented by the formula (3) is an alcohol compound represented by the formula (3-a):

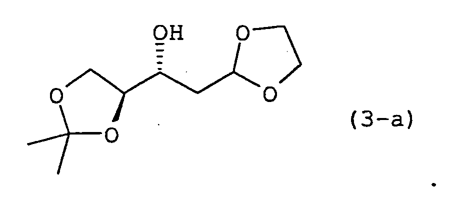

14. A production method of a furanose type 1-position hydroxyl-protected form of 2-deoxy-L-ribose, which comprises reacting a mixture of a furanose type 1-position hydroxyl-protected form of 2-deoxy-L-ribose and a furanose type 1-position hydroxyl-protected form of 2-deoxy-L-xylose with a ketone compound represented by the formula (5) 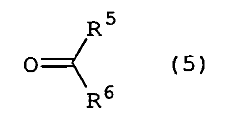 wherein R⁵ and R⁶ are each independently an alkyl group, an aralkyl group or an aryl group, or R⁵ and R⁶ in combination show a cyclic alkyl group, in the presence of an acid, to preferentially protect the 3-position and the 5-position hydroxyl groups of the furanose type 1-position hydroxyl-protected form of 2-deoxy-L-xylose and give a furanose type 2-deoxy-L-xylose compound represented by the formula (6) 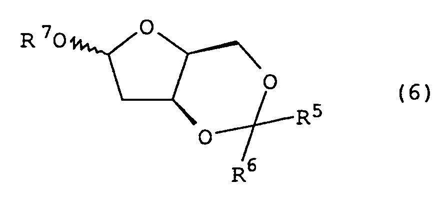 wherein R⁵ and R⁶ are as defined above and R⁷ is an alkyl group or an aralkyl group, and then separating an unreacted furanose type 1-position hydroxyl-protected form of 2-deoxy-L-ribose from the furanose type 2-deoxy-L-xylose compound represented by the formula (6).

15. The production method of claim 14, wherein the ketone compound represented by the formula (5) is acetone.

16. The production method of claim 14 or 15, wherein the separation comprises separation of water from an organic solvent.

17. A production method of a furanose type 2-deoxy-L-ribose represented by the formula (8) 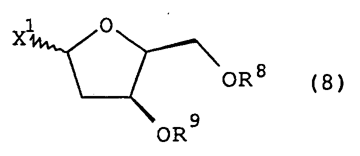 wherein X¹ is a leaving group, R⁸ and R⁹ are each independently a hydroxy-protecting group or R⁸ and R⁹ in combination show a hydroxyl-protecting group, which comprises protecting the 3-position and the 5-position hydroxyl groups of the furanose type 1-position hydroxyl-protected form of 2-deoxy-L-ribose obtained by the production method of any of claims 14 to 16, and then converting the protected 1-position hydroxyl group to a leaving group.

18. The production method of claim 17, wherein X¹ is a chlorine atom and R⁸ and R⁹ are p-toluoyl groups.

19. The production method of any of claims 14 to 18, wherein the mixture containing a furanose type 1-position hydroxyl-protected form of 2-deoxy-L-ribose and a furanose type 1-position hydroxyl-protected form of 2-deoxy-L-xylose are obtained by the production method of any of claims 4 to 6.

20. The production method of claim 19, wherein R¹ and R² are each independently a hydroxyl-protecting group, which can be deprotected by acid hydrolysis, or R¹ and R² in combination show a hydroxyl-protecting group, which can be deprotected by acid hydrolysis, and the deprotection of the hydroxyl group and the production of aldehyde by acid hydrolysis of Step (b) are simultaneously performed.

21. The production method of claim 20, wherein R¹ and R² in combination show a cyclic acetal type hydroxyl-protecting group, which can be deprotected by acid hydrolysis.

22. The production method of any of claims 19 to 21, wherein M is Li, MgX² or ZnX² (X² is a halogen atom).

23. The production method of claim 21, wherein the aldehyde compound represented by the formula (1) is an aldehyde compound represented by the formula (1-a): 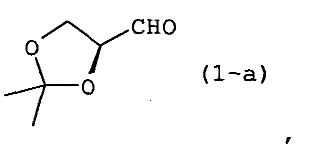 the organometallic compound represented by the formula (2) is an organometallic compound represented by the formula (2-a): 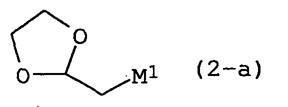 wherein M¹ is Li, MgX² or ZnX² (X² is a halogen atom), and the alcohol compound represented by the formula (3) is an alcohol compound represented by the formula (3-a):

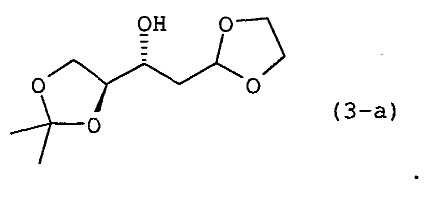

24. An alcohol compound represented by the formula (3): 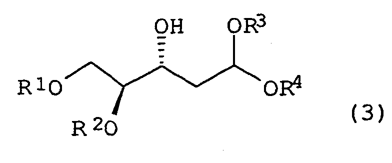 wherein R¹ and R² are each independently a hydroxyl-protecting group or R¹ and R² in combination show a hydroxyl-protecting group, R³ and R⁴ are each independently an alkyl group, an aralkyl group, an aryl group or a silyl group or R³ and R⁴ in combination show a cyclic alkyl group.

25. An alcohol compound represented by the formula (3-a):
